# EUROPÄISCHE PATENTSCHRIFT

(11) **EP 2 915 559 B1**
(45) Veröffentlichungstag und Bekanntmachung des Hinweises auf die Patenterteilung: **03.10.2018**
(21) Anmeldenummer: 15154830.2
(22) Anmeldetag: 12.02.2015
(51) Int. Cl.: A61N 1/08

(54) **IMPLANTIERBARES GERÄT**
IMPLANTABLE DEVICE
APPAREIL IMPLANTABLE

(30) Priorität: 06.03.2014 US 201461948576 P
(43) Veröffentlichungstag der Anmeldung: 09.09.2015
(73) Patentinhaber: BIOTRONIK SE & Co. KG, 12359 Berlin (DE)
(72) Erfinder: Stürmer, Uwe, 10119 Berlin (DE); Dörr, Thomas, 12437 Berlin (DE)
(74) Vertreter: Galander, Marcus

(56) Entgegenhaltungen:
- US-A1- 2014 058 481
- US-B1- 6 212 431

## Beschreibung

Die Erfindung betrifft eine Prüfeinrichtung für eine implantierbare Elektrodenleitung und ein implantierbares Gerät, das mit einer Elektrodenleitung verbunden oder zu verbinden ist. Die Erfindung betrifft insbesondere implantierbare Monitoring und/oder Therapiegeräte wie Neurostimulatoren, Herzschrittmacher, Kardioverter, Defibrillatoren oder der gleichen.
An implantierbaren Herzschrittmachern oder Defibrillatoren sind typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden.

Weist ein elektrischer Leiter der Elektrodenleitung einen Fehler auf, kann dies dazu führen, dass die Übertragung von Stimulationsimpulsen zu den Elektrodenpolen oder insbesondere auch die Übertragung elektrischer Signale von den Elektrodenpolen zum proximalen Ende der Elektrodenleitung nicht mehr zuverlässig sind. Bei einem implantierbaren Gerät in Form eines Herzschrittmachers kann dies zu Problemen beim Stimulieren, aber auch beim Sensing führen mit der Folge, dass beispielsweise intrakardiale Ereignisse gar nicht oder falsch erfasst werden. Derartige Fehler können sich erst im Laufe des Betriebs der Elektrodenleitung einstellen, also dann, wenn die Elektrodenleitung möglicherweise schon für einen längeren Zeitraum einem Patienten implantiert ist. Daher enthalten einige implantierbare Geräte eine Prüfeinrichtung, mit der sich Fehler an der Elektrodenleitung möglichst erkennen lassen.
Bekannte Ansätze zur Elektrodenfehlererkennung bedienen sich beispielsweise der Messung und Auswertung folgender Parameter:
- Elektrodenimpedanzen
- Signalamplituden
- Störsignalerkennung
- Reizschwellen
- diverse Plausibilitätsprüfungen
Die bekannten Methoden zur Elektrodenfehlererkennung zeigen jedoch immer wieder eine geringe Sensitivität und Spezifität. So können beispielsweise Isolationsdefekte an der Elektrodenleitung gar nicht mittels einer Impedanzmessung festgestellt werden, da die Impedanz der eigentlichen Elektrodenpole weit geringer ist als eine Verringerung der Impedanz infolge eines Isolationsdefektes, so dass der Isolationsdefekt bei der Impedanzprüfung nur zu einem unbedeutenden Abfall der gemessenen Impedanz führt. Ähnliches gilt für die anderen genannten Verfahren.

US 6,212,431 B1 offenbart eine Optimierung der Stromzufuhr zwischen der externen Spule und dem Implantat bei induktiver Kopplung. Diese Optimierung wird mit Hilfe eines Richtkopplers und eine Impedanz-Anpassung geschehen.

Der Erfindung liegt daher die Aufgabe zugrunde, eine Prüfeinrichtung zu schaffen, welche eine möglichst hohe Sensitivität und Spezifität in Bezug auf eine Elektrodenfehlererkennung besitzt.

Erfindungsgemäß wird diese Aufgabe durch eine Prüfeinrichtung für eine implantierbare Elektrodenleitung gelöst, die einen Richtkoppler oder einen Zirkulator zum Einkoppeln eines elektrischen Testsignals in wenigstens einen elektrischer Leiter der Elektrodenleitung sowie zum Auskoppeln des reflektierten Testsignals aus dem elektrischen Leiter der Elektrodenleitung aufweist. Ein derartiger Richtkoppler ermöglicht es, ein Testsignal, beispielsweise einen steilflankigen Spannungsimpuls, in einen elektrischen Leiter der Elektrodenleitung einzukoppeln und den zurücklaufenden reflektierten Spannungsimpuls (also das reflektierte Testsignal) mittels des Richtkopplers oder Zirkulators wieder aus dem elektrischen Leiter auszukoppeln. Die erfindungsgemäße Prüfeinrichtung nutzt den Effekt, dass Inhomogenitäten beispielsweise der Impedanz entlang eines elektrischen Leiters dazu führen, dass eine elektrische Welle, wie sie beispielsweise mit einem hochfrequenten Testsignal einhergeht, an Orten derartiger Inhomogenität zum Teil reflektiert wird. Während so also ein Teil der in Form des Testsignals in den in den elektrischen Leiter eingespeisten Welle von der Elektrodenleitung in die Umgebung abgegeben wird, wird ein anderer Teil dieser Welle an verschiedenen Orten der Elektrodenleitung teilreflektiert. Auf diese Weise ergibt sich ein charakteristisches reflektiertes Testsignal. Dieses charakteristische reflektierte Testsignal ist für eine intakte Elektrodenleitung anders als für eine fehlerbehaftete Elektrodenleitung, die beispielsweise einen Bruch eines elektrischen Leiters aufweist. Im letztgenannten Fall würde das Testsignal am Ort des Bruchs des elektrischen Leiters reflektiert werden, weil sich dort die Impedanz erhöht. Entsprechend wird ein größerer Teil des Testsignals bei einer derartigen fehlerbehafteten Elektrodenleitung früher reflektiert als bei einer intakten Elektrodenleitung. Dies führt dazu, dass sich ein anderes reflektiertes Testsignal ergibt als dies bei einer intakten Elektrodenleitung der Fall ist. Deswegen kann ein Elektrodenfehler anhand eines mittels des Richtkopplers oder Zirkulators in einen Leiter der Elektrodenleitung eingespeisten, in dem Leiter der Elektrodenleitung teilweise reflektierten und über den Richtkoppler oder Zirkulator wieder aus dem Leiter der Elektrodenleitung ausgespeisten Testsignal identifiziert werden.

Vorzugsweise weist die Prüfeinrichtung einen Testsignalgenerator auf, der mit dem Richtkoppler oder dem Zirkulator verbunden ist und der ausgebildet ist, ein über den Richtkoppler oder den Zirkulator in den elektrischen Leiter der Elektrodenleitung einzukoppelndes Testsignal zu erzeugen und abzugeben. Der Testsignalgenerator ist dabei vorzugsweise dazu ausgebildet, ein Testsignal mit hochfrequenten Signalanteilen, beispielsweisen einen steilflankigen Spannungsimpuls, zu erzeugen.

Außerdem weist die Prüfeinrichtung vorzugsweise eine Auswerteeinheit auf, die wenigstens indirekt mit dem Richtkoppler oder dem Zirkulator verbunden und die ausgebildet ist, ein mittels des Richtkopplers oder des Zirkulators aus dem elektrischen Leiter der Elektrodenleitung ausgekoppeltes reflektiertes Testsignal auszuwerten und in Abhängigkeit eines Ergebnisses der Auswertung ein einen Fehler der Elektrodenleitung anzeigendes Signal zu erzeugen und auszugeben. Die Auswerteeinheit ist dabei vorzugsweise ausgebildet, ein jeweils reflektiertes und ausgekoppeltes Testsignal mit einem Referenzsignal zu vergleichen, welches bei einer nachweislich intakten Elektrodenleitung aufgenommen wurde. Insbesondere ist die Auswerteeinheit vorzugsweise dazu ausgebildet, solche Eigenschaften des ausgekoppelten reflektierten Testsignals, wie Signalmaxima und die Zeitdauer zwischen Abgabe eines Testsignals und Erfassen eines Signalmaximums des reflektierten Testsignals, zu bestimmen und mit Referenzwerten zu vergleichen. Die Auswerteeinheit kann auch dazu ausgebildet sein, einen Morphologievergleich zwischen einem Referenzsignal für ein reflektiertes Testsignal und ein jeweiliges reflektiertes Testsignal durchzuführen. Insbesondere kann ein derartiger Morphologievergleich auch das Bestimmen der Zeitdauer zwischen Abgabe eines Testsignals und dem Erfassen bestimmter Merkmale des reflektierten Testsignals mit einschließen.

So ergibt sich eine Prüfeinrichtung zur verbesserten Erkennung von Elektrodenfehlern, wie Isolationsdefekten, Kurzschlüssen oder Leitungsbrüchen an implantierten Elektrodenleitungen, indem erfindungsgemäß die HF-Reflektionseigenschaften der Elektrodenleitung durch den Einsatz eines Richtkopplers im Implantat beurteilt werden.

Diese Prüfeinrichtung bietet den Vorteil, dass Defekte an einer Elektrodenleitung frühzeitig und zuverlässig erkannt werden können.

Der Richtkoppler oder Zirkulator ist vorzugsweise über einen hochfrequenztauglichen einpoligen Wechselschalter (Englisch: SPDT = Single Pole Double Throw) mit dem elektrischen Leiter der Elektrodenleitung verbunden. Auf diese Weise ist es möglich, den elektrischen Leiter von den typischen Bestandteilen eines implantierbaren Gerätes, wie beispielsweise den Bestandteilen einer Filterdurchführung, vollständig abzukoppeln und ausschließlich an den Testsignalgenerator und die Auswerteeinheit anzukoppeln. Eine Prüfung des elektrischen Leiters ist dann von den übrigen Komponenten eines implantierbaren Gerätes unbeeinträchtigt. Umgekehrt kann auf diese Weise vermieden werden, dass die Prüfeinrichtung den regulären Betrieb des implantierbaren Gerätes mit der Elektrodenleitung beeinträchtigt. Ein derartiger Wechselschalter kann beispielsweise als MEMS-Relais verwirklicht sein oder mit Hilfe von Transistoren. MEMS-Relais haben Vorteile in Bezug auf elektromagnetische Störungen (EMI) und sind daher bevorzugt.

Der Richtkoppler ist vorzugsweise in Microstrip-Technologie ausgeführt. Auf diese Weise kann der Richtkoppler besonders klein sein und leicht in eine Elektrodenleitung oder ein Anschlussgehäuse (Header) eines implantierbaren Gerätes integriert werden.

Vorzugsweise hat der Richtkoppler eine Bandbreite, die wenigstens einen Teil des Frequenzbereiches zwischen 400 MHz und 2,4 GHz umfasst, insbesondere den Frequenzbereich zwischen 400 MHz und 800 MHz. Im letztgenannten Frequenzbereich hat die zugehörige Welle eine Wellenlänge zwischen etwa 40 bis 70 cm, so dass die Wellenlänge oder die Hälfte oder ein Viertel davon in etwa der Länge des elektrischen Leiters der jeweiligen Elektrodenleitung entspricht.

Vorzugsweise ist die Prüfeinrichtung Teil eines implantierbaren Gerätes, das mit einer Elektrodenleitung verbunden oder zu verbinden ist. Dementsprechend wird die vorgenannte Aufgabe erfindungsgemäß auch durch ein implantierbares Gerät gelöst, das mit einer Elektrodenleitung verbunden oder zu verbinden ist und dass eine Prüfeinrichtung der hier beschrieben und beanspruchten Art aufweist. Die Prüfeinrichtung hat nämlich nicht nur den Vorteil, eine hohe Sensitivität und Spezifität bei der Elektrodenfehlererkennung zu besitzen, sie kann darüber hinaus auch so klein ausgeführt werden, dass sie sich in ein implantierbares Gerät, wie einen Herzschrittmacher/Defibrillator oder dergleichen, integrieren lässt oder sogar in eine Elektrodenleitung.

Vorzugsweise ist der Richtkoppler oder der Zirkulator über einen hochfrequenztauglichen einpoligen Wechselschalter mit der Elektrodenleitung oder einem Anschluss für eine Elektrodenleitung verbunden. Letztgenannte Ausführungsvariante ist vorteilhaft bei solchen implantierbaren Geräten, die einen Anschluss mit beispielsweise elektrischen Kontaktbuchsen für eine Elektrodenleitung aufweisen. In diesem Fall kann die Prüfeinrichtung oder zumindest der Richtkoppler oder der Zirkulator umschaltbar mit dem Anschluss für die Elektrodenleitung verbunden werden. Für den Normalbetrieb wird der Anschluss der Elektrodenleitung mittels des Wechselschalters mit den für den Normalbetrieb vorgesehenen elektrischen und elektronischen Komponenten des implantierbaren Gerätes verbunden. Auf diese Weise ergeben sich die vorgenannten Vorteile, nämlich insbesondere die Möglichkeit, auch im implantierbaren Gerät eine Filterdurchführung zu verwenden, ohne dass diese Filterdurchführung die Elektrodenfehlererkennung beeinträchtigt, weil sie nur im Normalbetrieb des implantierbaren Gerätes wirksam ist.

Bei einem implantierbaren Gerät, das ein Anschlussgehäuse (auch als Header bekannt) besitzt, in dem sich der Anschluss für eine Elektrodenleitung befindet, ist es vorteilhaft, wenn wenigstens der Richtkoppler oder der Zirkulator und gegebenenfalls auch der hochfrequenztaugliche einpolige Wechselschalter in dem Anschlussgehäuse angeordnet sind. Damit befinden sich der Richtkoppler oder der Zirkulator und gegebenenfalls der hochfrequenztaugliche einpolige Wechselschalter außerhalb des üblicherweise geschlossenen Metallgehäuses derartiger implantierbarer Geräte, und elektromagnetische Störungen innerhalb des geschlossenen metallischen Gehäuses des implantierbaren Gerätes werden wirksam vermieden. In Fortführung des letztgenannten Gedankens ist es vorteilhaft, bei einem Anschlussgehäuse, das wenigstens einen Anschluss für eine Elektrodenleitung und eine mit diesem verbundene Filterdurchführung aufweist, den Richtkoppler oder den Zirkulator gegebenenfalls über den hochfrequenztauglichen einpoligen Wechselschalter zwischen dem Anschluss für die Elektrodenleitung und der Filterdurchführung mit dem Anschluss für die Elektrodenleitung zu verbinden. Dies bedeutet, dass das Anschlussgehäuse wie üblich eine Filterdurchführung aufweist, die dazu dient, Signale aus der Elektrodenleitung in das geschlossene Metallgehäuse des implantierbaren Gerätes zu führen und umgekehrt Stimulationsimpulse, beispielsweise aus dem geschlossenen Metallgehäuse, in die Elektrodenleitung einzuspeisen. Indem die Prüfvorrichtung zwischen Filterdurchführung und dem eigentlichen Anschluss für die Elektrodenleitung mit diesem Anschluss verbunden ist, also beispielsweise der hochfrequenztaugliche einpolige Wechselschalter zwischen Filterdurchführung und Anschluss geschaltet ist, ist es möglich, die Prüfeinrichtung ganz außerhalb des geschlossenen metallischen Gehäuses zu realisieren.

Gemäß einer weiteren Ausführungsvariante ist es vorgesehen, einen im implantierbaren Gerät ohnehin für die Datenkommunikation vorgesehenen Transceiver auch als Testsignalgenerator zu nutzen. Dem liegt die Erkenntnis zugrunde, dass derartige Transceiver typischerweise in der Lage sind, hochfrequenzhaltige Signale zu erzeugen, die sich auch als Testsignale für die Elektrodenfehlererkennung eignen. Außerdem können derartige Transceiver das reflektierte ausgekoppelte Testsignal empfangen und verarbeiten. Dazu kann der für die Datenkommunikation vorgesehene Transceiver, der auch als Testsignalgenerator dient, mit der Auswerteeinheit der Prüfeinrichtung verbunden sein. Im Betrieb zur Elektrodenfehlererkennung erzeugt der Transceiver somit das Testsignal, welches über den Richtkoppler oder den Zirkulator und gegebenenfalls den hochfrequenztauglichen einpoligen Wechselschalter in die Elektrodenleitung eingespeist wird und in dieser teilweise reflektiert wird. Das zurücklaufende reflektierte Testsignal wird über den Zirkulator oder den Richtkoppler wieder aus der Elektrodenleitung ausgekoppelt und wieder dem Transceiver (der dann als Empfänger wirkt) zugeführt. Ein gegebenenfalls vom Transceiver demoduliertes reflektiertes Testsignal kann dann durch die Auswerteeinheit analysiert und/oder mit Referenzsignalen verglichen werden.

Gemäß einer weiteren Variante der Erfindung ist es vorgesehen, dass wenigstens der Richtkoppler oder auch der Zirkulator zum Einkoppeln eines elektrischen Testsignals in wenigstens einen elektrischen Leiter der Elektrodenleitung sowie zum Auskoppeln des reflektierten Testsignals aus dem elektrischen Leiter der Elektrodenleitung in die implantierbare Elektrodenleitung selbst integriert ist. Gegebenenfalls kann die gesamte Prüfeinrichtung in die Elektrodenleitung integriert sein. Auf jeden Fall ist es möglich, den Richtkoppler oder den Zirkulator, aber insbesondere den Richtkoppler, in die Elektrodenleitung zu integrieren.

Falls es sich um eine Elektrodenleitung zum Anschluss an ein implantierbares Gerät handelt, die dementsprechend einen üblichen Elektrodenstecker aufweist, ist es vorteilhaft, wenn eine Prüfeinrichtung der hier beschriebenen beanspruchten Art in den Elektrodenstecker integriert ist.

Erfindungsgemäß wird auch Verfahren zum Prüfen eines elektrischen Leiters einer implantierbaren Elektrodenleitung vorgeschlagen, welches die folgenden Verfahrensschritte aufweist:
- Einkoppeln eines Testsignals in den elektrischen Leiter
- Auskoppeln des in dem elektrischen Leiter reflektierten Testsignals aus dem elektrischen Leiter
- Auswerten des reflektierten Testsignals.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20;
- Fig. 2: ist eine Skizze zur Erläuterung des der Erfindung zugrundeliegenden Prinzips; und
- Fig. 3: zeigt ein Beispiel für eine erfindungsgemäße Prüfeinrichtung.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise einen Anschluss 16 mit Kontaktbuchsen zur Aufnahme von Steckkontakten einer Elektrodenleitung auf. Die Kontaktbuchsen besitzen elektrische Kontakte, die über entsprechende Leitungen und üblicherweise eine Filterdurchführung 18 mit einer in dem Gehäuse 12 des Herzstimulators 10 angeordneten Elektronik 210 verbunden sind.

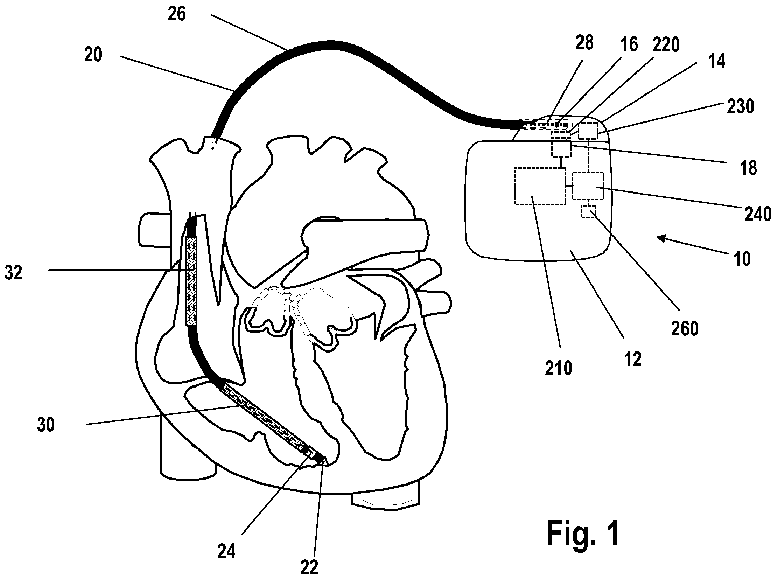

Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Elektrodenleitungsstecker 28 am proximalen Ende der Elektrodenleitung 20 elektrisch verbunden. Der Elektrodenleitungsstecker 28 besitzt elektrische Kontakte, die mit den elektrischen Kontakten der Kontaktbuchse des Anschlusses 16 im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

In Fig. 1 sind außerdem jene Bestandteile des implantierbaren Gerätes 10 dargestellt, die eine Prüfeinrichtung zur Elektrodenfehlererkennung darstellen. Zu diesen Bestandteilen zählt ein zwischen den Anschluss 16 und die Filterdurchführung 18 geschalteter, hochfrequenztauglicher einpoliger Wechselschalter 220, mit dessen Hilfe die Funktionsleiter der Elektrodenleitung 20 entweder mit der Filterdurchführung 18 elektrisch verbunden werden können oder alternativ mit einem Richtkoppler 230. Anstelle des Richtkopplers 230 kann auch ein Zirkulator vorgesehen sein.

Der Richtkoppler 230 ist so ausgebildet, dass ein Testsignal, das hochfrequente Signalanteile enthält, über den Wechselschalter 220 in einen oder mehrere der Leiter 26 der Elektrodenleitung 20 einkoppeln kann und in der Elektrodenleitung 20 reflektierte Signalanteile des Testsignals wieder auskoppeln kann. Das in den oder die Leiter 26 einzukoppelnde Testsignal wird von einem Testsignalgenerator 240 erzeugt und ausgegeben. Der Testsignalgenerator 240 ist im dargestellten Ausführungsbeispiel gleichzeitig ein Transceiver für eine drahtlose Datenkommunikation mit dem implantierbaren Gerät 10.

Rücklaufende (reflektierte) Signalanteile des Testsignals werden über den Wechselschalter 220 in den Richtkoppler 230 wiederum dem Transceiver 240 zugeführt, der seinerseits mit einer Auswerteeinheit 260 verbunden ist. Diese ist dazu ausgebildet, ein jeweils reflektiertes Testsignal (genauer die reflektierten zurücklaufenden Signalanteile des Testsignals) mit einem entsprechenden Referenzsignal zu vergleichen.

Für eine Prüfung der Elektrodenleitung 20 zum Zweck der Elektrodenfehlererkennung ist somit der Transceiver 240 als Testsignalgenerator und Auswerteeinheit über den Richtkoppler 230 und den entsprechend geschalteten Wechselschalter 220 mit jeweils wenigstens einem Leiter 26 der Elektrodenleitung 20 verbunden.

Im Normalbetrieb des implantierbaren Gerätes 10 ist der Wechselschalter 220 so geschaltet, dass der oder die Leiter 26 der Elektrodenleitung 20 mit der Filterdurchführung 18 und über diese mit der übrigen Elektronik 210 (beispielsweise einer Therapiesteuereinheit) verbunden ist. Da der Richtkoppler 230 in diesem Falle nicht mehr mit dem oder den Leiter(n) 26 der Elektrodenleitung 20 verbunden ist, kann die Prüfeinrichtung den regulären Betrieb des implantierbaren Gerätes 10 auch nicht beeinträchtigen.

Nur am Rande sei darauf hingewiesen, dass der Transceiver 240 auch mit der übrigen Elektronik 210 (Therapiesteuereinheit) verbunden ist, um eine Datenkommunikation, beispielsweise mit einem externen Gerät, in üblicher und an sich bekannter Weise zu erlauben.

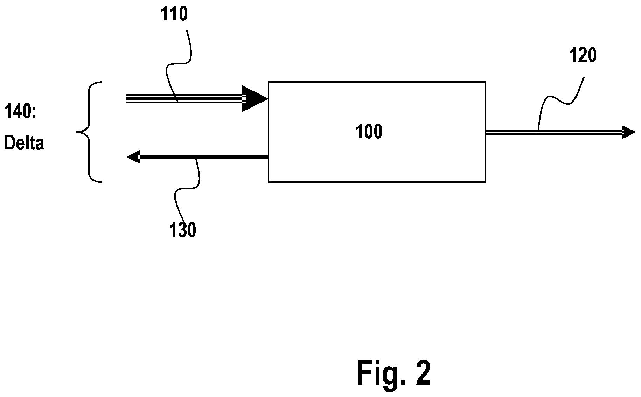

In Figur 2 ist das Grundprinzip der der Prüfeinrichtung durchzuführenden Messung dargestellt. Grundlage der Messung sind die Reflexionen auf Leitungen wie den Leitern 26 der Elektrodenleitung 20, die an jeder Diskontinuität auf der Elektrodenleitung 100 entstehen. Zur Messung wird die Elektrodenleitung 100 zunächst mit einem Testsignal 110 mit bekannter Frequenz, Amplitude und Phase beaufschlagt und so eine Welle entlang wenigstens eines Leiters 26 der Elektrodenleitung 100 erzeugt. Ein Teil 120 dieser Welle wird von der Elektrodenleitung in die Umgebung abgegeben und ein weiterer Teil 130 wird in der Elektrodenleitung an Diskontinuitäten derselben reflektiert. Dieser Teil 130 repräsentiert das zurücklaufende reflektierte Testsignal 130 und kann mit Hilfe des Richtkopplers 230 von der eingekoppelten Welle getrennt werden. Damit kann die Differenz 140 zwischen dem eingekoppelten Testsignal 110 und dem reflektierten, ausgekoppelten Testsignal 130 ermittelt und als Indikator für einen Elektrodenfehler ausgewertet werden.

Vorzugsweise wird für die Messung zur Elektrodenfehlererkennung ein Testsignal 110 erzeugt, das die Form einer Rechteckimpulsfolge hat, und in die zu untersuchende Elektrodenleitung eingekoppelt, um eine Unterscheidung zwischen dem Testsignal 110 und anderen Signalen wie beispielsweise Herzereignissen und Störsignalen zu ermöglichen.

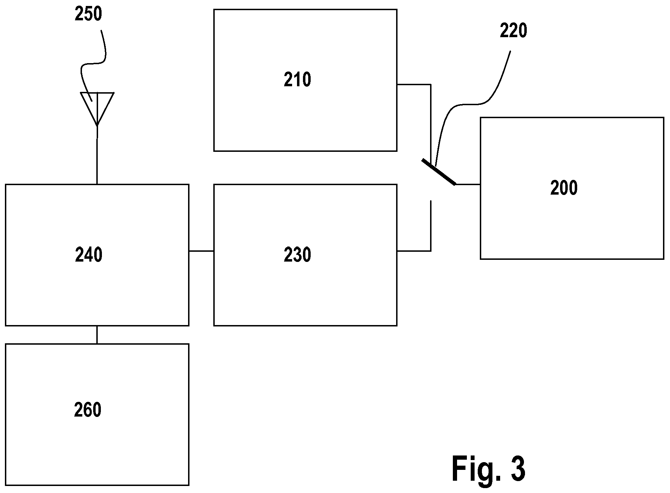

In Figur 3 ist ein mögliches Blockschaltbild für die vorgeschlagene Prüfeinrichtung zur Elektrodenfehlererkennung dargestellt. Die Elektrodenleitung 200 ist im Normalbetrieb immer mit der Therapiesteuereinheit 210 des implantierbaren Gerätes über einen hochfrequenztauglichen Wechselschalter 220 verbunden.

Für einen Test der Elektrodenleitung mittels einer Reflektionsmessung wird diese Elektrodenleitung nun mittels Wechselschalters 220 an mit Richtkoppler 230 verbunden. Dieser wiederum ist mit dem im implantierbaren Gerät vorhandenen RF-Transceiver 240 verbunden, der normalerweise mittels einer Antenne 250 der Kommunikation zwischen implantierbaren Gerät und externen Geräten dient. Für die Reflektionsmessung ist der RF-Transceiver 240 mit einem zusätzlichen Ausgang verbunden, um das Testsignal in den Richtkoppler 230 einzukoppeln und mit einem zusätzlichen Eingang versehen, um die reflektierte Welle (das reflektierte Testsignal) empfangen zu können. Ferner ist der RF-Receiver 240 mit einer Auswerteeinheit 260 ausgestattet, die der Differenzbildung zwischen eingestrahlter und empfangener Welle dient. In diesem Fall ist die Auswerteeinheit 260 indirekt mit dem Richtkoppler 230 verbunden. Die Auswerteeinheit 260 kann auch direkt mit dem Richtkoppler derart verbunden sein, das diese die zurücklaufende Welle (das reflektierte Testsignal) zur Analyse des Reflektionsverhaltens auf der Elektrodenleitung auswertet.

Der Richtkoppler 230 ist vorzugsweise ein Microstreifen-Richtkoppler im Anschlussgehäuse 14 des implantierbaren Gerätes 10, schaltbar auf die einzelnen zu untersuchenden Leiter 26 der Elektrodenleitung 20 durch den hochfrequenztauglichen einpoligen Wechselschalter 220, der beispielsweise entweder durch ein MEMS Relais (Vorteil bei EMI) oder Transistoren (Nachteilig bei EMI) realisiert sein kann. Mit dieser Anordnung können weiterhin die EMI Filter in den Durchführungen 18 verwendet werden.

Für eine Prüfung der Elektrodenleitung auf Elektrodenleitungsfehler wird die Prüfeinrichtung mittels Wechselschalters derart an die Elektrodenleitung geschaltet, dass die restliche Schaltung des implantierbaren Gerätes von dieser Prüfeinrichtung entkoppelt ist, d.h. die EMI-Kondensatoren und alle weiteren kapazitiven und induktiven Komponenten der Elektronik 210 des implantierbaren Gerätes beeinflussen die Reflektionsmessung nicht.

Eine derartige Prüfung der Elektrodenleitung erfolgt vorzugsweise in wiederkehrenden Intervallen, also zyklisch.

Vorzugsweise erfolgt die Auswertung der rücklaufenden Welle (also des reflektierten Testsignals) auf Basis eines Vergleiches mit eine, zuvor ermittelten Referenzsignal, das bei nachweislich intaktem Elektrodensystem aufgezeichnet wurde (z.B. Vergleich mit Referenz von der Implantation oder mit Trendwerten).

Die Information der Elektrodenfehlerauswertung wird vorzugsweise im implantierbaren Gerät gespeichert und bei der Nachsorge von einem externen Gerät, beispielsweise einem Programmiergerät ausgelesen und angezeigt. Alternativ oder zusätzlich kann die Information der Elektrodenfehlerauswertung über ein Fernüberwachungssystem zum Arzt übertragen werden. Alternativ oder zusätzlich ist es möglich in Abhängigkeit Information der Elektrodenfehlerauswertung die Betriebsart oder andere Betriebsparameter des Implantates automatisch anzupassen und/oder ein Signal an den Patienten (Ton/Vibration o.ä.) abzugeben und/oder eine automatische Umkonfiguration der genutzten Elektrodenleitungen auszulösen, z.B. eine Umschaltung von bipolar zu unipolar oder umgekehrt. Die hier genannten Varianten ergeben entsprechende vorteilhafte Ausgestaltungen des implantierbaren Gerätes.

Optional kann die Analyse des reflektierten Testsignals auch der für die Diagnostik einer Elektrodendislokation genutzt werden, weil die Reflektionseigenschaften der Elektrodenleitung auch davon abhängen welche elektrischen Eigenschaften die unmittelbare Umgebung eines jeweiligen Elektrodenpols am distalen Ende eines jeweiligen elektrischen Leiters der Elektrodenleitung hat. Entsprechend ist ein implantierbares Gerät bevorzugt, welches dazu ausgebildet ist, ein reflektiertes Testsignal hinsichtlich solcher Signalmerkmale auszuwerten, die einen Hinweis auf die Umgebung eines jeweiligen Elektrodenpols geben, z.B. hinsichtlich seiner Wandständigkeit.

Bezüglich der Anordnung der Prüfeinrichtung oder von Teilen der Prüfeinrichtung sind folgende Varianten vorteilhaft:
- Wenigstens Teile der Prüfeinrichtung sind im Gehäuse 12 des implantierbaren Gerätes 10 untergebracht.
- Die in Prüfeinrichtung ist im Anschlussgehäuse 14 des implantierbaren Gerätes 10 untergebracht, so dass nach wie vor gefilterte Durchführungen eingesetzt werden können.
- Die Prüfeinrichtung ist innerhalb des Elektrodenleitungssteckers 28 oder innerhalb der Elektrodenleitung 12 untergebracht.
- Der Richtkoppler ist integraler Bestandteil der Elektrodenleitung 20 oder des Elektrodenleitungssteckers 28.

Die Bandbreite des Richtkopplers ist vorzugsweise so gewählt, dass für die Signalgenerierung und die Analyse der zurücklaufenden Welle der für die RF-Kommunikation ohnehin vorhandene Transceiver genutzt werden kann. Bevorzugte Frequenzen sind dabei das MICS-Band, das ISM-Band (speziell auch das BlueTooth LE Band) oder ein GSM/UMTS-Band.

Die Bandbreite des Richtkopplers 230 ist kann zusätzlich oder alternativ auch auf die zu erwartenden Elektrodenleiterlängen eingestellt sein (λ; λ/2; λ/4) und ist z.B. in einem Bereich von 400-800 MHz entsprechend einer Wellenlänge λ zwischen ungefähr 40 cm bis 70 cm.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Ebenso kommen für die Anwendung der Erfindung weitere, mit Elektroden- oder Sensorleitungen verbundene elektronische Implantate, wie zum Beispiel Neurostimulatoren oder Drucksensorimplantate in Betracht.

### Bezugszeichenliste

- 10: Herzstimulator
- 12: Gehäuse
- 14: Anschlussgehäuse
- 16: Anschluss (Kontaktbuchsen) für Elektrodenleitung
- 18: Filterdurchführung
- 20: Elektrodenleitung
- 22: Spitzen- oder Tipelektrode
- 24: Ringelektrode
- 26: elektrischer Leiter
- 28: Elektrodenleitungsstecker
- 30, 32: Elektrodenpole
- 100: elektrischer Leiter
- 110: eingekoppeltes Testsignal
- 120: in die Umgebung austretender Teil der Welle
- 130: reflektiertes Testsignal
- 200: Elektrodenleitung
- 210: Elektronik (Therapiesteuereinheit)
- 220: Wechselschalter
- 230: Richtkoppler
- 240: Testsignalgenerator (Transceiver)
- 250: Antenne
- 260: Auswerteeinheit

## Patentansprüche

1. Prüfeinrichtung für eine implantierbare Elektrodenleitung (20) mit wenigstens einem elektrischen Leiter (26; 100), **gekennzeichnet durch** einen Richtkoppler (230) oder einen Zirkulator zum Einkoppeln eines elektrischen Testsignals in wenigstens einen elektrischen Leiter (26; 100) der Elektrodenleitung (20) sowie zum Auskoppeln des reflektierten Testsignals aus dem elektrischen Leiter (26; 100) der Elektrodenleitung (20; 200), **gekennzeichnet durch** einen Testsignalgenerator (240), der mit dem Richtkoppler (230) oder dem Zirkulator verbunden ist und der ausgebildet ist, ein über den Richtkoppler (230) oder den Zirkulator in den elektrischen Leiter (26; 100) der Elektrodenleitung (20; 200) einzukoppelndes Testsignal zu erzeugen und abzugeben, und durch eine Auswerteeinheit (260), die wenigstens indirekt mit dem Richtkoppler (230) oder dem Zirkulator verbunden und ausgebildet ist, ein mittels des Richtkopplers (230) oder des Zirkulators aus dem elektrischen Leiter (26; 100) der Elektrodenleitung (20; 200) ausgekoppeltes reflektiertes Testsignal auszuwerten und in Abhängigkeit eines Ergebnisses der Auswertung ein einen Fehler der Elektrodenleitung (20; 200) anzeigendes Signal zu erzeugen und auszugeben.

2. Prüfeinrichtung nach Anspruch 1, **dadurch gekennzeichnet, dass** das Testsignal (110) hochfrequente Signalanteile enthält und insbesondere ein steilflankiger Spannungsimpuls ist.

3. Prüfeinrichtung nach wenigstens einem der Ansprüche 1 bis 2, **dadurch gekennzeichnet, dass** der Richtkoppler (230) oder der Zirkulator über einen hochfrequenztauglichen einpoligen Wechselschalter (220) mit dem elektrischen Leiter (26; 100) der Elektrodenleitung (20; 200) verbunden ist.

4. Prüfeinrichtung nach wenigstens einem der Ansprüche 1 bis 3, **dadurch gekennzeichnet, dass** der Richtkoppler (230) in Microstrip-Technologie ausgeführt ist.

5. Prüfeinrichtung nach wenigstens einem der Ansprüche 1 bis 4, **dadurch gekennzeichnet, dass** der Richtkoppler (230) eine Bandbreite hat, die wenigstens einen Teil des Frequenzbereichs zwischen 400 MHz und 2,4 GHz umfasst, insbesondere den Frequenzbereich zwischen 400 MHz und 800 MHz.

6. Implantierbares Gerät, das mit einer Elektrodenleitung (20; 200) verbunden oder zu verbinden ist, **gekennzeichnet durch** eine Prüfeinrichtung gemäß wenigstens eines der Ansprüche 1 bis 5.

7. Implantierbares Gerät nach Anspruch 6, **dadurch gekennzeichnet, dass** der Richtkoppler (230) oder der Zirkulator über einen hochfrequenztauglichen einpoligen Wechselschalter (220) mit der Elektrodenleitung (20; 200) oder einem Anschluss (16) für eine Elektrodenleitung (20; 200) verbunden ist.

8. Implantierbares Gerät nach Anspruch 6 oder 7, **dadurch gekennzeichnet, dass** das implantierbare Gerät (10) ein Anschlussgehäuse (14) aufweist und wenigstens der Richtkoppler (230) oder der Zirkulator und ggf. der hochfrequenztaugliche einpolige Wechselschalter (220) in dem Anschlussgehäuse angeordnet sind.

9. Implantierbares Gerät gemäß Anspruch 8, **dadurch gekennzeichnet, dass** das Anschlussgehäuse (14) wenigstens einen Anschluss (16) für eine Elektrodenleitung (20; 200) und eine mit diesem verbundene Filterdurchführung (18) aufweist und der Richtkoppler (230) oder der Zirkulator ggf. über den hochfrequenztauglichen einpoligen Wechselschalter (220) zwischen dem Anschluss (16) für eine Elektrodenleitung (20; 200) und der Filterdurchführung (18) mit dem Anschluss (16) für eine Elektrodenleitung (20; 200) verbunden ist.

10. Implantierbares Gerät nach wenigstens einem der Ansprüche 6 bis 9, **dadurch gekennzeichnet, dass** der Testsignalgenerator (240) ein Transceiver (240) für eine Datenkommunikation des implantierbaren Gerätes (10) ist.

11. Implantierbare Elektrodenleitung (20; 200) als Teil eines implantierbaren Gerätes oder zum Anschluss an ein implantierbares Gerät, mit einem Elektrodenleitungsstecker (28) an einem proximalen Ende der Elektrodenleitung (20; 200), **dadurch gekennzeichnet, dass** der Elektrodenleitungsstecker (28) eine Prüfeinrichtung gemäß wenigstens eines der Ansprüche 1 bis 5 enthält.

12. Verfahren zum Prüfen eines elektrischen Leiters einer implantierbaren Elektrodenleitung (20; 200), mit den Schritten:
- Einkoppeln eines Testsignals in den elektrischen Leiter (26; 100),
- Auskoppeln des in dem elektrischen Leiter (26; 100) reflektierten Testsignals aus dem elektrischen Leiter, und
- Auswerten des reflektierten Testsignals.

## Claims

1. A test apparatus for an implantable electrode line (20) comprising at least one electrical conductor (26; 100), **characterised by** a directional coupler (230) or a circulator for coupling an electrical test signal into at least one electrical conductor (26; 100) of the electrode line (20) and for decoupling the reflected test signal from the electrical conductor (26; 100) of the electrode line (20; 200), **characterised by** a test signal generator (240), which is connected to the directional coupler (230) or the circulator and which is designed to generate and to deliver a test signal to be coupled into the electrical conductor (26; 100) of the electrode line (20; 200) via the directional coupler (230) or the circulator, and by an evaluation unit (260), which is connected at least indirectly to the directional coupler (230) or the circulator and is designed to evaluate a reflected test signal decoupled by means of the directional coupler (230) or the circulator from the electrical conductor (26; 100) of the electrode line (20; 200) and to generate and to output a signal indicating a fault of the electrode line (20; 200) in accordance with a result of the evaluation.

2. The test apparatus according to claim 1, **characterised in that** the test signal (110) contains high-frequency signal components and in particular is a steep voltage pulse.

3. The test apparatus according to at least one of claims 1 to 2, **characterised in that** the directional coupler (230) or the circulator is connected to the electrical conductor (26; 100) of the electrode line (20; 200) via a single-pole, double-throw switch (220) suitable for high frequencies.

4. The test apparatus according to at least one of claims 1 to 3, **characterised in that** the directional coupler (230) is formed on the basis of microstrip technology.

5. The test apparatus according to at least one of claims 1 to 4, **characterised in that** the directional coupler (230) has a bandwidth which comprises at least part of the frequency range between 400 MHz and 2.4 GHz, in particular the frequency range between 400 MHz and 800 MHz.

6. An implantable device which is connected or is to be connected to an electrode line (20; 200), **characterised by** a test apparatus according to at least one of claims 1 to 5.

7. The implantable device according to claim 6, **characterised in that** the directional coupler (230) or the circulator is connected to the electrode line (20; 200) or to a terminal (16) for an electrode line (20; 200) via a single-pole, double-throw switch (220) suitable for high frequencies.

8. The implantable device according to claim 6 or 7, **characterised in that** the implantable device (10) comprises a terminal housing (14) and at least the directional coupler (230) or the circulator and, where applicable, the single-pole, double-throw switch (220) suitable for high frequencies are arranged in the terminal housing.

9. The implantable device according to claim 8, **characterised in that** the terminal housing (14) comprises at least one terminal (16) for an electrode line (20; 200) and a filter feedthrough (18) connected thereto, and the directional coupler (230) or the circulator is connected, between the terminal (16) for an electrode line (20; 200) and the filter feedthrough (18), to the terminal (16) for an electrode line (20; 200), where applicable, via the single-pole, double-throw switch (220) suitable for high frequencies.

10. The implantable device according to at least one of claims 6 to 9, **characterised in that** the test signal generator (240) is a transceiver (240) for data communication of the implantable device (10).

11. An implantable electrode line (20; 200) as part of an implantable device or for connection to an implantable device, comprising an electrode line plug (28) at a proximal end of an electrode line (20; 200), **characterised in that** the electrode line plug (28) contains a test apparatus according to at least one of claims 1 to 5.

12. A method for testing an electrical conductor of an implantable electrode line (20; 200) comprising the following steps:
- coupling a test signal into the electrical conductor (26; 100);
- decoupling the test signal reflected in the electrical conductor (26; 100) from the electrical conductor; and
- evaluating the reflected test signal.

## Revendications

1. Dispositif de test pour une ligne d'électrode (20) implantable avec au moins un conducteur électrique (26 ; 100), **caractérisé par** un coupleur directionnel (230) ou un circulateur pour coupler un signal de test électrique dans au moins un conducteur électrique (26 ; 100) de la ligne d'électrode (20), ainsi que pour découpler le signal de test réfléchi du conducteur électrique (26 ; 100) de la ligne d'électrode (20 ; 200), **caractérisé par** un générateur de signal de test (240) qui est relié avec le coupleur directionnel (230) ou le circulateur et qui est conçu pour générer et émettre un signal de test se couplant dans le conducteur électrique (26 ; 100) de la ligne d'électrode (20 ; 200) par le biais du coupleur directionnel (230) ou du circulateur, et **caractérisé par** une unité de traitement (260) qui est reliée au moins indirectement avec le coupleur directionnel (230) ou avec le circulateur et est conçue pour évaluer un signal de test réfléchi découplé du conducteur électrique (26 ; 100) de la ligne d'électrode au moyen du coupleur directionnel (230) ou du circulateur, et pour générer et émettre un signal indiquant un défaut de la ligne d'électrode (20 ; 200) en fonction d'un résultat de l'évaluation.

2. Dispositif de test selon la revendication 1, **caractérisé en ce que** le signal de test (110) contient des parties de signaux de haute fréquence et est notamment une impulsion de tension à forte inclinaison.

3. Dispositif de test selon au moins une des revendications 1 à 2, **caractérisé en ce que** le coupleur directionnel (230), ou le circulateur, est relié avec le conducteur électrique (26 ; 100) de la ligne d'électrode (20 ; 200) par le biais d'un commutateur de sélection (220) mono polaire compatible avec les hautes fréquences.

4. Dispositif de test selon au moins une des revendications 1 à 3, **caractérisé en ce que** le coupleur directionnel (230) est conçu avec la technologie de la ligne micro-ruban, microstrip.

5. Dispositif de test selon au moins une des revendications 1 à 4, **caractérisé en ce que** le coupleur directionnel (230) a une largeur de bande qui comprend au moins une partie du domaine de fréquences entre 400 MHz et 2,4 GHz, en particulier, le domaine de fréquences entre 400 MHz et 800 MHz.

6. Appareil implantable qui est relié ou est à relier avec une ligne d'électrode (20 ; 200), **caractérisé par** un dispositif de test selon au moins l'une des revendications 1 à 5.

7. Appareil implantable selon la revendication 6, **caractérisé en ce que** le coupleur directionnel (230), ou le circulateur, est relié avec la ligne d'électrode (20 ; 200) ou un connecteur (16) pour une ligne d'électrode (20 ; 200) par le biais d'un commutateur de sélection (220) mono polaire compatible avec les hautes fréquences.

8. Appareil implantable selon la revendication 6 ou 7, **caractérisé en ce que** l'appareil implantable (10) présente un boîtier de raccordement (14) et qu'au moins le coupleur directionnel (230) ou le circulateur, et éventuellement, des commutateurs de sélection (220) mono polaires compatibles avec les hautes fréquences sont disposés dans le boîtier de raccordement.

9. Appareil implantable selon la revendication 8, **caractérisé en ce que** le boîtier de raccordement (14) présente au moins un connecteur (16) pour une ligne d'électrode (20 ; 200) et une traversée filtrante (18) reliée avec celui-ci, et le coupleur directionnel (230), ou le circulateur, est relié et avec le connecteur (16) pour une ligne d'électrode (20 ; 200), éventuellement par le biais des commutateurs de sélection (220) mono polaires compatibles avec les hautes fréquences entre le connecteur (16) pour une ligne d'électrode (20 ; 200) et la traversée filtrante (18).

10. Appareil implantable selon au moins l'une des revendications 6 à 9, **caractérisé en ce que** le générateur de signal de test (240) est un émetteur-récepteur (240) pour une communication de données de l'appareil implantable (10).

11. Ligne d'électrode (20; 200) implantable sous forme d'une partie d'un appareil implantable ou pour le raccordement à un appareil implantable avec une prise de ligne d'électrode (28) à une extrémité proximale de la ligne d'électrode (20 ; 200), **caractérisée en ce que** la fiche d'alimentation de ligne d'électrode (28) contient un dispositif de test selon au moins l'une des revendications 1 à 5.

12. Procédé de test d'un conducteur électrique d'une ligne d'électrode implantable (20 ; 200) avec les étapes :
- de couplage d'un signal de test dans le conducteur électrique (26, 100),
- de découplage du signal de test réfléchi dans le conducteur électrique (26 ; 100) hors du conducteur électrique, et
- d'évaluation du signal de test réfléchi.
